(19) Europäisches Patentamt
European Patent Office
Office européen des brevets

(11) **EP 2 602 282 B1**

(12) **EUROPÄISCHE PATENTSCHRIFT**

(45) Veröffentlichungstag und Bekanntmachung des Hinweises auf die Patenterteilung:
**29.10.2014 Patentblatt 2014/44**

(51) Int Cl.:
***C08J 5/18*** *(2006.01)*   ***C08K 5/00*** *(2006.01)*
*C08K 5/103* *(2006.01)*   *C08L 53/02* *(2006.01)*

(21) Anmeldenummer: **11191956.9**

(22) Anmeldetag: **05.12.2011**

(54) **Elastische Folie für den Windelbereich**

Elastic film for the nappy area

Film élastique pour la partie de couche

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**12.06.2013 Patentblatt 2013/24**

(73) Patentinhaber: **Mondi Gronau GmbH**
**48599 Gronau (DE)**

(72) Erfinder:
• **Sollmann, Henner**
**48599 Gronau (DE)**

• **Baldauf, Georg, Dipl.-Ing.**
**48366 Laer (DE)**

(74) Vertreter: **Albrecht, Rainer Harald**
**Andrejewski - Honke**
**Patent- und Rechtsanwälte**
**An der Reichsbank 8**
**45127 Essen (DE)**

(56) Entgegenhaltungen:
**EP-A1- 2 325 255      WO-A1-00/66662**
**WO-A1-2008/087675   WO-A1-2009/152870**

EP 2 602 282 B1

**Beschreibung**

Die Erfindung betrifft eine elastische Folie für den Windelbereich, insbesondere für elastische Windelverschlusselemente, mit einer durch Extrusion einer Polymerzusammensetzung hergestellten elastischen Schicht, wobei die Polymerzusammensetzung eine Mischung eines Styrolblockcopolymers und eines Weichmachers aufweist.

Die Folie wird insbesondere zur Herstellung von Windelverschlusselementen in Form von Verschlusslaschen oder elastischen Windelohren eingesetzt. Sie weist zumindest eine elastische Schicht eines thermoplastischen Elastomers auf. Thermoplastische Elastomere sind Kunststoffe, die sich bei Raumtemperatur vergleichbar den klassischen Elastomeren verhalten, sich jedoch unter Wärmezufuhr plastisch verformen lassen und somit ein thermoplastisches Verhalten zeigen.

Die erfindungsgemäße Folie umfasst eine elastische Schicht die Styrolblockcopolymere enthält. Dabei handelt es sich um Zweiphasensysteme, die aus einer elastischen Weichphase und einer thermoplastischen Hartphase bestehen. Die Styrolblöcke bestehen aus Styroleinheiten, die vorzugsweise mit Polybutadiensegmenten verbunden sind. Bei Styrolblockpolymeren werden Mineralöle als Weichmacher eingesetzt, um die Härte herabzusetzen. Die Weichmacheraufnahme ist unter anderem von der Kornstruktur des Polymergranulats abhängig. Bei herkömmlichen Weichmachern handelt es sich um Weichmacheröle, die auf Basis von Erdöl gewonnen werden. Sie werden auch als Weißöle bezeichnet.

Erdöl ist ein fossiler Rohstoff, der immer knapper wird. Zudem führen seine Förderung und Verarbeitung zu Risiken für die Umwelt und leisten einen Beitrag zur Klimaerwärmung.

Aufgabe der Erfindung ist es, eine elastische Folie für den Windelbereich und ein Verfahren zu ihrer Herstellung anzugeben, die eine nachhaltige Rohstoffbasis aufweist. Zudem soll die Folie preiswert sein und sich durch vorteilhafte Produkteigenschaften, beim Einsatz als Windelverschlusselemente auszeichnen. Auch soll eine hohe Verbraucherakzeptanz gewährleistet sein.

Diese Aufgabe wird erfindungsgemäß dadurch gelöst, dass der Weichmacher zu mehr als 50 Gew.-Gew. ausRapsöl besteht.

Durch den Ersatz zumindest eines Teils des Weißöl durch Rapsöl, ein pflanzlich basiertes Öl wird der Anteil an endlichen Ressourcen, wie beispielsweise Erdöl, vermindert. Zudem verbessert sich die $CO_2$-Bilanz. Dies trägt zu einer nachhaltigen Produktion bei. Bei hohen Rohölpreisen haben pflanzlich basierte Öle gegenüber Weißölen zudem einen Kostenvorteil. Auch weisen sie bessere Geruchseigenschaften auf.

Der Austausch eines Mineralöl basierten Weißöls durch ein Pflanzenöl wirkt sich auf die Elastizität der Folie nicht nachteilig aus. Eine elastische Folie, die ein pflanzliches Öl als Weichmacher enthält, ist tendenziell etwas weicher alls eine mit Weißöl gefertigte Folie. Dabei zeichnen sich erfindungsgemäß unter Verwendung von Rapsöl gefertigte Folien nach einer elastischen Dehnung auch durch eine niedrige Restdehnung aus.

Dabei besteht der Weichmacher zu mehr als 50 Gew.-% aus Rapsöl, vorzugsweise zu mehr als 80 Gew.-%. Bei einer besonders vorteilhaften Ausführung der Erfindung besteht der Weichmacher vollständigaus Rapsöl.

Kunststoffe, in denen Pflanzenöle als Weichmacher verwendet werden, sind bekannt. So wird in der WO 00/66662 eine thermoplastische Elastomerzusammensetzung beschrieben, die Styrolblockcopolymere und ein Pflanzenöl als Plastifizierungsmittel enthält. Die Kunststoffe werden zur Herstellung von Formteilen eingesetzt. Eine Anwendung von Pflanzenölen als Weichmacher bei elastischen Folien, die für Windeln geeignet sind und beispielsweise als elastische Windelverschlusselemente appliziert werden, wurde bisher nicht in Betracht gezogen. Dies liegt nicht zuletzt an den besonders hohen Qualitätsanforderungen, die vom Verbraucher an die Produkt- und Gebrauchseigenschaften von Babyprodukten gestellt werden. Dabei beeinflussen bereits geringe Abweichungen in der Polymerzusammensetzung die Qualität dieser Folien. Erstaunlicherweise wurde festgestellt, dass pflanzliche Öle als Weichmacher einen positiven Einfluss auf die Produkteigenschaften elastischer Folien für Windelverschlusselemente haben können. Dazu ist jedoch eine gezielte Auswahl an pflanzlichen Ölen erforderlich, da ansonsten die komplexen Prozesse der Folienherstellung für den Windelbereich negativ beeinflusst werden.

Vorzugsweise werden pflanzliche Öle mit einer Iodzahl von kleiner als 150, vorzugsweise kleiner als 100, eingesetzt. Die Bestimmung der Iodzahl ist eine analytische Methode zur Erfassung von Doppelbindungen in Ölen, die nach einer normierten Vorschrift erfolgt. Dabei ist die Iodzahl die Masse an Iod in Gramm, die von 100 g Substanz gebunden wird. Bei der Auswahl pflanzlicher Öle erweisen sich Öle mit einer Iodzahl von kleiner als 150, vorzugsweise kleiner als 100, als besonders geeignet für die Herstellung von Folien für den Windelbereich, da diese eine besondere Stabilität gegenüber Sauerstoff und UV-Strahlung, sowie eine hohe thermische Stabilität aufweisen.

Als ein ergänzendes oder alternatives Auswahlkriterium für den Einsatz von pflanzlichen Ölen zur Produktion von Folien für den Windelbereich erweist sich der Rauchpunkt. Auch der Rauchpunkt wird nach einer normierten Vorschrift bestimmt. Dabei handelt es sich um die Temperatur, bei der sich das Öl in Gegenwart von Luft unter Rauchbildung zersetzt. Zur Folienherstellung für den Windelbereich werden pflanzliche Öle ausgewählt, die einen Rauchpunkt von mehr als 180 °C, insbesondere mehr als 200 °C, aufweisen. Dabei eignen sich besonders raffinierte Öle, da diese, gegenüber nicht raffinierten Ölen, höhere Rauchpunkte aufweisen.

Als ein weiteres Kriterium zur Auswahl geeigneter pflanzlicher Öle wird ergänzend oder alternativ die kinematische Viskosität herangezogen. In Versuchen hat sich gezeigt, dass diese einen entscheidenden Einfluss auf die Qualität der hergestellten Folien für den Windelbereich hat. Dabei haben sich pflanzliche Öle als besonders vorteilhaft erwiesen, die eine kinematische Viskosität bei 20 °C von weniger als 85 $\frac{mm^2}{s}$ und mehr als 60 $\frac{mm^2}{s}$ aufweisen.

Unter Berücksichtung der zuvor aufgeführten Auswahlkriterien und unter zusätzlicher Beachtung von Geruchseigenschaften und Farbbeeinflussung erweist sich der Einsatz von raffiniertem Rapsöl als Weichmacheröl als besonders vorteilhaft für die Herstellung von Folien für den Windelbereich.

Insbesondere die folgenden pflanzlichen Öle sind als Weichmacher in der Polymerzusammensetzung für die Extrusion einer elastischen Schicht geeignet: Rapsöl; Sojaöl, Sonnenblumenöl, Maiskeimöl, Baumwollsaatöl, Reiskeimöl, Erdnussöl, Olivenöl, Safloröl. Bevorzugt sind raffinierte Pflanzenöle.

Das Rapsöl kann einzeln oder in Mischungen mit den vorgenannten Ölen eingesetzt werden. Insbesondere können sie auch als Gemisch mit Weißöl, d. h. einem auf Erdölbasis gewonnenen Weichmacheröl, verwendet werden, wobei der Anteil des Rapsöls erfindungsgemäß mindestens 50 Gew.-% bezogen auf die Gesamtmenge des in der Polymerzusammensetzung eingesetzten Weichmachers beträgt.

Die Herstellung der Folie für den Windelbereich erfolgt in mehreren Schritten. Zunächst wird die Mischung aus dem Styrolblockcopolymer und dem pflanzlichen Weichmacheröl, Rapsöl, hergestellt. Dabei wird das Weichmacheröl auf ein Granulat des Styrolblockcopolymers gegeben. Der Anteil an Weichmacher in der Mischung beträgt dabei zweckmäßig zwischen 10 Gew.-% und 60 Gew.-%. Vorzugsweise enthält die Mischung einen Weichmacheranteil zwischen 20 Gew. % und 40 Gew.-%.

Als Styrolblockcopolymer eignet sich ein hochmolekulares lineares Styrol-Butadien-Styrol (SBS)-Blockpolymer. Die Mischung wird gerührt bis das Pflanzenöl in die Polymerkörner eingezogen ist.

In einem nächsten Schritt werden zu dieser Mischung weitere Komponenten gegeben. Der Anteil der Mischung an der Polymerzusammensetzung beträgt dabei vorzugsweise mehr als 30 Gew.-%, insbesondere mehr als 40 Gew.-% und weniger als 60 Gew.-%, insbesondere weniger als 50 Gew.-%. Die Polymerzusammensetzung, d.h. die Mischung und die weiteren Komponenten, kann mittels Zweiwellenextruder bzw. Compounder verarbeitet werden.

Bei einer vorteilhaften Variante der Erfindung enthält die Polymerzusammensetzung zusätzlich zu dem in der Mischung bereits vorhandenen Styrolblockcopolymeranteil, einen Anteil eines linearen SBS-Blockcopolymers von mehr als 30 Gew.-% und weniger als 60 Gew. %. Zudem erweist es sich als günstig, wenn die Polymerzusammensetzung mehr als 5 Gew.-% und weniger als 30 Gew.-% eines Polystyrols enthält. Bei einer vorteilhaften Variante sind außerdem Thermostabilisatoren enthalten. Außerdem können weitere Zusatzstoffe wie Farbpigmente enthalten sein.

Bei einer besonders vorteilhaften Ausführung der Erfindung weist die für den Windelbereich hergestellte Folie mindestens eine mit der Polymerzusammensetzung coextrudierte Deckschicht auf, wobei vorzugsweise die aus der Polymerzusammensetzung hergestellte elastische Schicht zwischen zwei Deckschichten eingebettet ist. Dadurch wird ein Verkleben der Folie beim Aufrollen vermieden. Als Material für die Deckschicht haben sich Polymere aus der Gruppe Polyethylen, Polyethylen-Copolymere, Polypropylen, Polypropylen-Copolymere oder einer Mischung einer dieser Polymere als günstig erwiesen. Die polyolefinische Außenschicht ist dabei vorzugsweise dünn im Vergleich zu der elastischen Schicht. Die Dicke der Außenschicht beträgt weniger als 20 %, insbesondere weniger als 10 %, der Dicke der elastischen Schicht.

Bei einer besonders vorteilhaften Variante ist auf die Folie zumindest eine textile Außenschicht aufkaschiert. Die Verbindung zwischen der textilen Außenschicht und der Folie kann auf verschiedene Weise erfolgen. Eine bevorzugte Ausführung sieht vor, dass die textile Außenschicht mit der Folie verklebt wird, wobei der Klebstoff flächig oder in einem punkt- oder streifenförmigen Muster appliziert wird. Vorzugsweise wird ein Nonwoven als textile Außenschicht eingesetzt.

Für Anwendungen im Windelbereich, insbesondere für Windelverschlusselemente, ist es vorteilhaft, wenn die elastische Folie in einer Richtung zugfest und nur in Querrichtung elastisch dehnbar ist. Zur Verbesserung ihrer Elastizität wird die Folie vor ihrer Verwendung mechanisch aktiviert. Bei der Aktivierung wird sie in der gewünschten Zugrichtung, üblicherweise CD ("Cross-Direction") mittels Streckwalzen gedehnt.

Weitere Merkmale und Vorteile der Erfindung ergeben sich aus der Beschreibung von Ausführungsbeispielen.

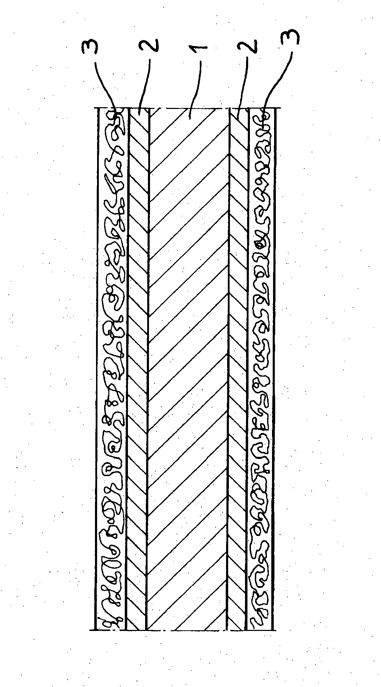

Die Figur zeigt einen Schnitt durch eine Folie, welche die Basis für die Herstellung von Windelverschlusselementen bildet. Die Folie hat einen mehrschichtigen Aufbau mit einer elastischen mittleren Schicht 1, die zwischen zwei Deckschichten 2 eingebettet ist. Die Gesamtstärke der Folie beträgt 60 μm. Die Deckschichten 2 bestehen aus einem Polyethylen/Polypropylen-Gemisch und haben eine Dicke von jeweils 5 μm. Auf jede Deckschicht 2 ist eine textile Außenschicht 3 aus Nonwoven aufkaschiert.

Bei der Herstellung der Folie wird in einem ersten Schritt eine Mischung aus einem Styrolblockcopolymer und Rapsöl hergestellt. Als weitere Weichmacheröle kommen insbesondere folgende Pflanzenöle in Betracht: Sonnenblumenöl, Maiskeimöl, Sojaöl, Baumwollsaatöl, Reiskeimöl, Erdnussöl, Olivenöl und Safloröl. Bevorzugt sind raffinierte Pflanzenöle.

Das Weichmaleröl wird nach mehreren Kriterien ausgewählt, damit es den hohen Anforderungen an eine Folie für Windelverschlusselemente genügt. In der Tabelle 1 sind Stoffeigenschaften aufgeführt, die als Auswahlkriterien für den Einsatz des Pflanzenöls als Weichmacher herangezogen werden.

Tabelle 1: Auswahlkriterien für unterschiedliche Weichmacheröle

|  | Rapsöl | Sonnen-blumenöl | Mais-keimöl | Sojaöl | Baum-wollsaatöl | Reis-keimöl | Erdnussöl | Olivenöl | Safloröl |
|---|---|---|---|---|---|---|---|---|---|
| Jodzahl | 105-115 | 120-140 | 107-130 | 120-141 | 105-115 | 92-115 | 80-107 | 75-90 | 138-152 |
| Kinematische Viskosität bei 20°C (in mm²/sec) | 74 | 65,8 | 64,1 | 63,5 | 65 | 76 | 80,4 | 83,8 | 65,8 |
| Farbe | braungelb | gelb | hell- bis goldgelb | hellgelb | hellgelb | hellgelb | gelblich | hellgelb | hellgelb |
| Geruch | schwach nach Pflanzenöl | schwach nach Pflanzenöl | schwach nach Pflanzenöl | schwach nach Pflanzenöl | schwach nach Pflanzenöl | schwach nach Pflanzenöl | schwach nach Pflanzenöl | schwach nach Pflanzenöl | schwach nach Pflanzenöl |
| Schmelzpunkt | -5°C | -18°C bis -16°C | -18°C bis -10°C | -15°C bis -8°C | -2°C | -5°C bis 10°C | -2°C | Ca. 0°C | -5°C |
| Rauchpunkt | >200°C | ca. 220°C | ca. 220°C | ca. 234°C | 216°C | Ca. 254°C | 200-235°C | 230°C | >200°C |

Bei der Herstellung von Folien für Windelverschlusselemente erweisen sich Öle mit folgenden Stoffeigenschaften als besonders günstig:

- einer Jodzahl von kleiner als 150, vorzugsweise kleiner als 120,
- einem Rauchpunkt von mehr als 180 °C, insbesondere mehr als 200 °C,
- einer kinematischen Viskosität bei 20 °C von weniger als 85 $\frac{mm^2}{s}$ und mehr als 60 $\frac{mm^2}{s}$,
- einem angenehmen Geruch,
- einer möglichst hellen Farbe.

Bei Anwendung dieser Kriterien zur Auswahl eines Weichmacheröls erweist sich unter anderem raffiniertes Rapsöl als günstig.

In einem ersten Herstellungsschritt wird eine Mischung aus einem Styrolblockcopolymer und einem Pflanzenöl hergestellt. In Tabelle 2 sind Mischungen eines Stryolblockcopolymers in Kombination mit unterschiedlichen Pflanzenölen aufgeführt. Als Styrolblockcopolymer wird ein hochmolekulares lineares Styrol-Butadien-Styrol (SBS) eingesetzt.

Tabelle 2: Unterschiedliche Mischungen

| Rohstoff [Gew.-%] | V1 | V2 | V3 | V4 | V5 | V6 |
|---|---|---|---|---|---|---|
| Hochmolekulares lineares SBS-Blockcopolymer | 70 | 70 | 70 | 70 | 70 | 70 |
| Weißöl | 30 | - | - | - |  |  |
| Erdnussöl | - | 30 | - | - |  |  |
| Maiskeimöl | - | - | 30 | - |  |  |
| Rapsöl | - | - | - | 30 |  |  |
| Sojaöl | - | - | - | - | 30 | - |
| Sonnenblumenöl | - | - | - | - | - | 30 |

Die Mischungen V2 bis V6 haben einen Anteil eines Styrolblockcopolymers von 70 Gew.-% in Kombination mit 30 Gew.-% Weichmacheröl. Bei der ersten Mischung V1 handelt es sich um eine herkömmliche Mischung mit einem Weißöl. Diese dient als Referenz.

Die Tabelle 3 zeigt, dass bei den Versuchen mit den pflanzlichen Ölen ein geringeres Drehmoment bei der Herstellung der Mischung unter Verwendung eines Kneters auftritt und somit ein geringerer Energieaufwand bei der Herstellung erforderlich ist.

4

Tabelle 3

| | maximales Drehmoment (in Nm) | Drehmoment am Versuchsende (in Nm) | maximale Temperatur (in°C) Versuchsende |
|---|---|---|---|
| Weißöl | 10,8 | 5,2 | 187,9 |
| Erdnussöl | 8,3 | 4,9 | 186,3 |
| Baumwollsaatöl | 8,1 | 4,7 | 186,7 |
| Maiskeimöl | 8,0 | 4,5 | 187,6 |
| Olivenöl | 8,0 | 3,6 | 186,1 |
| Rapsöl | 8,3 | 4,2 | 187,2 |
| Reiskeimöl | 7,7 | 4,3 | 186,7 |
| Safloröl | 7,0 | 4,1 | 187,1 |
| Sojaöl | 7,3 | 4,3 | 186,7 |
| Sonnenblumenöl | 7,9 | 3,8 | 187,4 |

Nach Erzeugung der Mischung wird in einem nächsten Schritt die Polymerzusammensetzung hergestellt.

In Tabelle 4 sind die Bestandteile einer Polymerzusammensetzung dargestellt.

Tabelle 4: Polymerzusammensetzung

| Rohstoff | Menge (in Gew.-%) |
|---|---|
| Mischung aus Styrolblockcopolymer und Weichmacheröl (Blend 70/30) | 50 |
| Lineares SBS-Blockcopolymer | 40 |
| Polystyrol | 10 |

Die Polymerzusammensetzung enthält 50 Gew.-% der Mischung eines Styrolblockcopolymers und eines Weichmachers. Zu der Mischung wird ein lineares Styrol-Butadien-Styrol (SBS)-Copolymer gegeben, dessen Anteil an der Polymerzusammensetzung 40 Gew.-% beträgt. Zudem wird 10 Gew.-% Polystyrol zugegeben. Thermostabilisatoren sind ebenfalls vorhanden.

Tabelle 5 zeigt das Drehmoment und den Materialdruck während der Compoundierung der Polymerzusammensetzung in einem Doppelschneckenextruder.

Tabelle 5: Drehmoment und Materialdruck während der Compoundierung

| Compound | Drehmoment (Skalierung in %) | Materialdruck (in bar) |
|---|---|---|
| Weißöl | 48 | 22 |
| Erdnussöl | 45 | 20 |
| Sonnenblumenöl | 44 | 21 |
| Maiskeimöl | 44 | 19 |
| Rapsöl | 44 | 19 |
| Sonnenblumenöl | 43 | 19 |
| Sojaöl | 42 | 20 |

Es zeigt sich, dass das Drehmoment und der Materialdruck bei der Compoundierung der Polymermischung beim Einsatz von pflanzlichen Ölen geringer sind als beim Einsatz von Weißöl. Somit ist auch hier ein geringerer Energieaufwand bei der Verarbeitung erforderlich.

Das Compound wird für die Kernschicht einer mehrschichtigen, durch Coextrusion gefertigten Folie eingesetzt. Die mehrschichtige Folie weist auf beiden Seiten der Kernschicht eine 5 μm dicke Außenschicht aus einem Polyethylen/Polypropylen-Gemisch auf und hat eine Gesamtdicke von 60 μm. Die unter Verwendung der zuvor beschriebenen Compounds hergestellten mehrschichtigen Folien werden einem Geruchsvergleich unterzogen. Die Versuche werden in Anlehnung an die DIN 10955 und an die VDA 270 durchgeführt. Die Bewertung erfolgt nach den Intensität von 1 bis 10 und nach der Wirkung des Geruchs von -4 bis +4. In Tabelle 6 sind Ergebnisse der Geruchsprüfung aufgeführt.

## Tabelle 6: Geruchstest

|  | Geruchswirkung | Geruchsintensität |
|---|---|---|
| Weißöl | -1 | 5 |
| Maiskeimöl | 0,1 | 3,4 |
| Rapsöl | 0,6 | 3,8 |
| Sonnenblumenöl | 0,6 | 4,6 |
| Sojaöl | 0,8 | 4,4 |

Die mit den ausgewählten Pflanzenölen hergestellten Folien schneiden sowohl bei der Geruchswirkung als auch bei der Geruchsintensität besser ab als die mit Weißöl produzierten Folien.

Elastische Folien, die erfindungsgemäß unter Verwendung von raffinierten Pflanzenölen als Weichmacher hergestellt werden, sind für den Windelbereich geeignet und können als Windelverschlusselemente eingesetzt werden.

**Patentansprüche**

1. Elastische Folie für den Windelbereich, insbesondere für elastische Windelverschlusselemente, mit einer durch Extrusion einer Polymerzusammensetzung hergestellten elastischen Schicht (1), wobei die Polymerzusammensetzung eine Mischung eines Styrolblockcopolymers und eines Weichmachers aufweist, **dadurch gekennzeichnet, dass** der Weichmacher zu mehr als 50 Gew.-% aus Rapsöl besteht.

2. Elastische Folie nach Anspruch 1, **dadurch gekennzeichnet, dass** das Rapsöl raffiniert ist.

3. Elastische Folie nach Anspruch 1 oder 2, **dadurch gekennzeichnet, dass** das Rapsöl

   - eine Jodzahl von kleiner als 150, insbesondere kleiner als 120, und/oder
   - einen Rauchpunkt von mehr als 180 °C, insbesondere mehr als 200 °C und/oder
   - eine kinematische Viskosität bei 20 °C von weniger als 85 $\frac{mm^2}{s}$ und mehr als 60 $\frac{mm^2}{s}$

   aufweist.

4. Elastische Folie nach einem der Ansprüche 1 bis 3, **dadurch gekennzeichnet, dass** der Weichmacher vollständig aus Rapsöl besteht.

5. Elastische Folie nach einem der Ansprüche 1 bis 4, **dadurch gekennzeichnet, dass** der Anteil an Weichmacher in der Mischung mehr als 10 Gew.-%, insbesondere mehr als 20 Gew. % und weniger als 60 Gew.-%, insbesondere weniger als 40 Gew.-%, beträgt.

6. Elastische Folie nach einem der Ansprüche 1 bis 5, **dadurch gekennzeichnet, dass** der Anteil der Mischung in der Polymerzusammensetzung mehr als 30 Gew.-%, insbesondere mehr als 40 Gew.-% und weniger als 60 Gew.-%, insbesondere weniger als 50 Gew.-% beträgt.

**7.** Elastische Folie nach einem der Ansprüche 1 bis 6, **dadurch gekennzeichnet, dass** die Polymerzusammensetzung zusätzlich zu dem in der Mischung vorhandenen Styrolblockcopolymeranteil einen Anteil eines linearen SBS-Blockcopolymers von 30 Gew.-% bis 60 Gew.-% aufweist.

**8.** Elastische Folie nach einem der Ansprüche 1 bis 7, **dadurch gekennzeichnet, dass** die Polymerzusammensetzung mehr als 5 Gew.-% und weniger als 30 Gew.-% eines Polystyrols aufweist.

**9.** Elastische Folie nach einem der Ansprüche 1 bis 8, **dadurch gekennzeichnet, dass** die Folie mindestens eine mit der Polymerzusammensetzung coextrudierte Deckschicht aufweist.

**10.** Elastische Folie nach Anspruch 9, **dadurch gekennzeichnet, dass** die coextrudierte Deckschicht aus einem Polymer aus der Gruppe Polyethylen, Polyethylen-Copolymere, Polypropylen, Polypropylen-Copolymere oder einer Mischung einer dieser Polymere besteht.

**11.** Verfahren zur Herstellung einer elastischen Folie für den Windelbereich, insbesondere für elastische Windelverschlusselemente, nach einem der Ansprüche 1 bis 10, wobei zumindest eine elastische Schicht aus einer Polymerzusammensetzung extrudiert wird, die eine Mischung eines Styrolblockcopolymers und eines Weichmachers enthält, **dadurch gekennzeichnet, dass** ein Weichmacher eingesetzt wird, der zu mehr als 50 Gew. % Rapsöl besteht.

**12.** Verfahren nach Anspruch 11, **dadurch gekennzeichnet, dass** die Polymerzusammensetzung mit mindestens einer Deckschicht coextrudiert wird.

**13.** Verfahren nach Anspruch 12, **dadurch gekennzeichnet, dass** die Polymerzusammensetzung mit mindestens einer Deckschicht aus einem Polymer der Gruppe Polyethylen, Polyethylen-Copolymer, Polypropylen, Polypropylen-Copolymer oder einer Mischung dieser Polymere coextrudiert wird.


**Claims**

**1.** The elastic foil for the nappy area, particularly for elastic nappyclosure elements, with an elastic layer produced by extruding a polymer composition (1), whereby the polymer composition exhibits a mixture of styrol block copolymers and a softening agents, is **characterised in that** the softening agent is composed of more than 50% by weight of rapeseed oil.

**2.** Elastic foil according to claim 1, **characterised in that** rapeseed oil is refined.

**3.** Elastic foil according to claim 1 or 2, **characterised in that** rapeseed oil has an

- iodine value of less than 150, in particular less than 120 and/or
- a smoke point of more than 180 °C, in particular more than 200 °C and/or
- a kinematic viscosity at 20 °C of less than 85 $mm^2$/s and more than 60 $mm^2$/s.

**4.** Elastic foil according to one of the claims 1 to 3, **characterised in that** the softening agent is completely made of rapeseed oil.

**5.** Elastic foil according to one of the claims 1 to 4, **characterised in that** the share of softening agent in the mixture amounts to more than 10% by weight, in particular more than 20 % by weight and less than 60% by weight, in particular more than less than 40% by weight.

**6.** Elastic foil according to one of the claims 1 to 5, **characterised in that** the proportion of the mixture in the polymer composition amounts to more than 30% by weight, in particular more than more than 40% by weight and less than 60% by weight, in particular more than less than 50% by weight.

**7.** Elastic foil according to one of the claims 1 to 6, **characterised in that** in addition to the styrol block polymer share present in the mixture, the polymer composition also has a share of a linear SBS block copolymer of 30% by weight to 60% by weight.

**8.** Elastic foil according one of the claims 1 to 7, **characterised in that** the polymer composition exhibits polystyrene of more than 5% by weight and less than 30% by weight.

9. Elastic foil according to one of the claims 1 to 8, **characterised in that** the foil exhibits at least one cover layer co-extruded with the polymer composition.

10. Elastic foil according to claim 9, **characterised in that** the co-extruded cover layer is made of a polymer from the group polyethylene, polyethylene copolymer, polypropylene, polypropylene copolymer or a mixture of one of these polymers.

11. Method of producing an elastic foil for the nappy area, particularly for the elastic nappy closure elements, according to one of the claims 1 to 10, whereby at least one elastic layer is extruded from a polycomposition, which contains a mixture of a styrol blockpolymer and a softening agent, **characterised in that** a softening agent is used which is composed of more than 50% by weight of rapeseed oil.

12. Method according to claim 11, **characterised in that** the polymer-composition is co-extruded with at least one cover layer.

13. Method according to claim 12, **characterised in that** that polymer-composition is co-extruded with at least one cover layer from a polymer of the group polyethylene, polyethylene copolymer, polypropylene, polypropylene copolymer or a mixture of one of these polymers.


## Revendications

1. Film élastique pour les couches, en particulier pour les éléments de fermeture élastiques des couches, avec une couche élastique (1) produite par une composition de polymères extrudés, celle-ci présentant un mélange d'un copolymère styrène séquencé avec un adoucisseur, **caractérisé par le fait que** l'adoucisseur est constitué d'huile de colza en ce qui concerne 50 % du poids.

2. Film élastique conformément à la revendication 1, **caractérisé par le fait que** l'huile de colza est raffinée.

3. Film élastique conformément à la revendication 1 ou 2, **caractérisé par le fait que** l'huile de colza présente

   - un indice d'iode inférieur à 150, notamment inférieur à 120, et/ou
   - un point de dégagement de fumée supérieur à 180 °C, notamment supérieur à 200 °C, et/ou
   - une viscosité cinématique à 20 °C inférieure à 85 mm$^2$/s et supérieure à 60 mm$^2$/s.

4. Film élastique conformément à l'une des revendications 1 à 3, **caractérisé par le fait que** l'adoucisseur est entièrement constitué d'huile de colza.

5. Film élastique conformément à l'une des revendications 1 à 4, **caractérisé par le fait que** la part de l'adoucisseur dans le mélange est supérieure à 10 %, notamment supérieure à 20 % du poids, et est inférieure à 60 %, notamment inférieure à 40 % du poids.

6. Film élastique conformément à l'une des revendications 1 à 5, **caractérisé par le fait que** la part du mélange dans la composition de polymères est supérieure à 30 %, notamment supérieure à 40 % et est inférieure à 60 % du poids, notamment inférieure à 50 % de celui-ci.

7. Film élastique conformément à l'une des revendications 1 à 6, **caractérisé par le fait que** la composition de polymères présente une part d'un copolymère styrène séquencé SBS linéaire de 30 à 60 % du poids en plus de la part de copolymères styrènes séquencés dans le mélange.

8. Film élastique conformément à l'une des revendications 1 à 7, **caractérisé par le fait que** la composition de polymères représente plus de 5 % et moins de 30 % du poids d'un polystyrène.

9. Film élastique conformément à l'une des revendications 1 à 8, **caractérisé par le fait que** le film présente au moins une couche de couverture coextrudée avec la composition de polymères.

**10.** Film élastique conformément à la revendication 9, **caractérisé par le fait que** la couche de couverture coextrudée est composée d'un polymère du groupe polyéthylènes, polyéthylène-copolymères, polypropylènes, polypropylène-copolymères ou d'un mélange d'un de ces polymères.

**11.** Procédé pour la fabrication d'un film élastique pour les couches, en particulier pour les éléments de fermeture élastiques des couches conformément à l'une des revendications 1 à 10, une couche élastique au moins étant extrudée d'une composition de polymères qui contient un mélange d'un polymère styrène séquencé et d'un adoucisseur, **caractérisé par le fait qu'**un adoucisseur est ajouté, lequel est constitué d'huile de colza en ce qui concerne plus de 50 % du poids.

**12.** Procédé conformément à la revendication 11, **caractérisé par le fait que** la composition de polymères est coextrudée avec au moins une couche de couverture.

**13.** Procédé conformément à la revendication 12, **caractérisé par le fait que** la composition de polymères est coextrudée avec au moins une couche de couverture composée d'un polymère du groupe polyéthylènes, polypropylène-copolymères, polypropylènes, polypropylènes-copolymères ou d'un mélange de ces polymères.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0066662 A